# EUROPÄISCHE PATENTSCHRIFT

(11) **EP 2 895 597 B1**
(45) Veröffentlichungstag und Bekanntmachung des Hinweises auf die Patenterteilung: **12.04.2017**
(21) Anmeldenummer: 13759235.8
(22) Anmeldetag: 09.09.2013
(51) Int. Cl.: C12N 1/26

(54) **VERFAHREN ZUR FERMENTATIVEN PRODUKTION VON L-CYSTEIN UND DERIVATEN DIESER AMINOSÄURE**
METHOD FOR THE FERMENTATIVE PRODUCTION OF L-CYSTEINE AND DERIVATIVES OF SAID AMINO ACID
PROCÉDÉ DE PRODUCTION FERMENTATIVE DE L-CYSTÉINE ET DE DÉRIVÉS DE CET ACIDE AMINÉ

(30) Priorität: 17.09.2012 DE 102012216527
(43) Veröffentlichungstag der Anmeldung: 22.07.2015
(73) Patentinhaber: Wacker Chemie AG, 81737 München (DE)
(72) Erfinder: DASSLER, Tobias, 81825 München (DE); LEINFELDER, Walfred, 84556 Kastl (DE); WICH,Günter, 81377 München (DE)
(74) Vertreter: Potten, Holger
(86) Internationale Anmeldenummer: PCT/EP2013/068629
(87) Internationale Veröffentlichungsnummer: WO 2014/040955

(56) Entgegenhaltungen:
- EP-A1- 1 382 684
- NAKAMORI ET AL: "overproduction of L-cysteine and L-cystine by Escherichia coli strains with a genetically altered serine acetyltransferase", APPLIED AND ENVIRONMENTAL MICROBIOLOGY, AMERICAN SOCIETY FOR MICROBIOLOGY, US, Bd. 64, Nr. 5, 1. Mai 1998 (1998-05-01), Seiten 1607-1611, XP002115630, ISSN: 0099-2240

## Beschreibung

Die Erfindung betrifft ein Verfahren zur fermentativen Produktion von L-Cystein und den Derivaten dieser Aminosäure L-Cystin und 2-Methyl-thiazolidin-2,4-dicarbonsäure.

L-Cystin ist ein Disulfid, das bei der Oxidation von zwei Molekülen L-Cystein entsteht. Diese Reaktion ist reversibel, was bedeutet, dass L-Cystin durch Reduktion wieder in L-Cystein überführt werden kann.

2-Methyl-thiazolidin-2,4-dicarbonsäure (Thiazolidin) ist das Kondensationsprodukt aus L-Cystein und Pyruvat, das in einer rein chemischen Reaktion gebildet wird (US5972663A). Diese Reaktion ist ebenfalls reversibel. So kann das Thiazolidin z.B. im Sauren bei erhöhter Temperatur wieder in seine Ausgangsbestandteile zerlegt werden.

Die Aminosäure L-Cystein ist von wirtschaftlicher Bedeutung. Sie wird beispielsweise als Lebensmittelzusatzstoff (insbesondere in der Backmittelindustrie), als Einsatzstoff in der Kosmetik, sowie als Ausgangsprodukt für die Herstellung von Pharmawirkstoffen (insbesondere N-Acetyl-Cystein und S-Carboxy- Methyl-Cystein) verwendet.

L-Cystein nimmt in allen Organismen eine Schlüsselposition im Schwefelmetabolismus ein und wird in der Synthese von Proteinen, Glutathion, Biotin, Liponsäure, Methionin und anderen schwefelhaltigen Metaboliten verwendet. Zudem dient L-Cystein als Vorläufer für die Biosynthese von Coenzym A. Die Biosynthese von L-Cystein wurde in Bakterien, insbesondere in Enterobakterien, detailliert untersucht und ist ausführlich in Kredich (1996, Biosynthesis of cysteine, p. 514-527. In F. C. Neidhardt, R. Curtiss III, J. L. Ingraham, E. C. C. Lin, K. B. Low, B. Magasanik, W. S. Reznikoff, M. Riley, M. Schaechter, and H. E. Umbarger (ed.), Escherichia coli and Salmonel-1a: cellular and molecular biology, 2nd ed. ASM Press, Washington, D.C.) beschrieben.

Ein wichtiger Vorläufer aus dem Zentralstoffwechsel bei der L-Cystein-Biosynthese ist 3-Phosphoglycerat, ein Intermediat der Glykolyse. L-Cystein wird daher zusammen mit L-Serin und Glycin zur Phosphoglycerat-Familie gezählt. Die proteinogenen Aminosäuren L-Methionin, L-Isoleucin und L-Threonin gehören hingegen zur Oxalacetat-Familie, da der gemeinsame Vorläufer aus dem Zentralstoffwechsel das Zitronensäurezyclus-Intermediat Oxalacetat ist. Ausgehend von einer Kohlenstoffquelle wie z.B. Glucose erfolgt der Stofffluss über diese beiden unterschiedlichen Äste des assimilatorischen Aminosäure-Metabolismus weitestgehend unabhängig voneinander. Allerdings sind bestimmte Interaktionen von Zwischen- oder Endprodukten der Phosphoglycerat-Familie mit einzelnen Biosynthese-Schritten der Oxalacetat-Familie bekannt. So wurde beschrieben, dass die Homoserin-Dehydrogenase I, das *thrA*-Genprodukt, welches die Reduktion von L-Aspartat-Semialdehyd zu L-Homoserin katalysiert, sowohl durch L-Serin als auch durch L-Cystein inhibiert werden kann (Hama et al., 1991, J. Biochem. 109, 604-8; Datta, 1967, Biochemistry 58, 635-41). Darüber hinaus ist bekannt, dass die assimilatorische Threonin-Deaminase IlvA durch L-Cystein gehemmt wird (Harris, 1981, J. Bacteriol. 145, 1031-35). Ferner kann L-Serin - analog zu L-Threonin - als Substrat des IlvA-Genprodukts dienen (Rasko et al., 1971, Eur. J. Biochem. 21, 424-27).

In verschiedenen Patenten ist zwar beschrieben, dass L-Isoleucin dem Kulturmedium bei der fermentativen Produktion von Aminosäuren der Phosphoglycerat-Familie zugesetzt werden kann (EP1233067, EP1382684), allerdings wird L-Isoleucin darin nur als eine von verschiedenen möglichen Zusätzen genannt. Dass L-Isoleucin möglicherweise einen positiven Einfluss auf die Aminosäure-Produktion haben könnte, wird jedoch an keiner Stelle gezeigt oder nahegelegt.

In Daßler et al. (2000, Mol. Microbiol. 36: 1101-1112) ist der Zusatz der Aminosäuren L-Methionin, L-Isoleucin und L-Leucin zum Medium der Vorkultur (jeweils 0,3 g/L) bei der fermentativen Produktion von Aminosäuren der Phosphoglycerat-Familie beschrieben. Dem Medium der Hauptkultur im Fermenter wurden diese Aminosäuren allerdings nicht zugesetzt. Auch hier findet sich kein Hinweis darauf, dass die Aminosäure-Supplementation des Vorkulturmediums einen positiven Einfluss auf die Cystein- oder Acetylserin-Produktion haben könnte.

Neben der klassischen Gewinnung von L-Cystein mittels Extraktion aus keratinhaltigem Material wie Haaren, Borsten, Hörnern, Hufen und Federn oder mittels Biotransformation durch enzymatische Umsetzung von Vorstufen wurde vor einigen Jahren auch ein Verfahren zur fermentativen Herstellung von L-Cystein entwickelt. Der Stand der Technik bezüglich der fermentativen Gewinnung von L-Cystein mit Mikroorganismen ist z.B. in US6218168B1, US5972663A, US20040038352A1, CA2386539A1, US20090053778A1 und US20090226984A1 beschrieben. Als bakterielle Wirtsorganismen werden dabei u.a. Stämme der Gattung Corynebakterium sowie Vertreter aus der Familie der Enterobacteriaceae, wie z.B. *Escherichia cola* oder *Pantoea ananatis* verwendet.

Neben der klassischen Vorgehensweise, um durch Mutation und Selektion zu verbesserten L-Cystein-Produzenten zu gelangen, wurden auch gezielt genetische Veränderungen an den Stämmen vorgenommen, um eine effektive L-Cystein-Überproduktion zu erreichen.

So führte das Einbringen eines cysE-Allels, das für eine Serin-O-Acetyl-Transferase mit einer verminderten Feedback-Hemmung durch L-Cystein kodiert, zu einer Steigerung der Cystein-Produktion (US6218168B1; Nakamori et al., 1998, Appl. Env. Microbiol. 64: 1607-1611). Durch ein feedback-resistentes CysE-Enzym wird die Bildung von O-Acetyl-L-Serin, der direkten Vorstufe von L-Cystein, weitgehend vom L-Cystein-Spiegel der Zelle entkoppelt.

O-Acetyl-L-Serin wird aus L-Serin und Acetyl-CoA gebildet. Daher ist die Bereitstellung von L-Serin in ausreichender Menge für die L-Cystein-Produktion von großer Bedeutung. Dies kann durch das Einbringen eines serA-Allels, das für eine 3-Phosphoglycerat-Dehydrogenase mit einer verminderten Feedback-Hemmbarkeit durch L-Serin kodiert, erreicht werden. Dadurch wird die Bildung von 3-Hydroxypyruvat, einer Vorstufe von L-Serin, weitgehend vom L-Serin-Spiegel der Zelle entkoppeln- Beispiele für derartige SerA-Enzyme sind in EP0620853 und US2005009162A2 beschrieben.

Darüber hinaus ist bekannt, dass die L-Cystein-Ausbeute in der Fermentation dadurch erhöht werden kann, dass Gene abgeschwächt oder zerstört werden, die für L-Cystein-abbauende Enzyme kodieren, wie z.B. die Tryptophanase TnaA oder die Cystathionine-β-Lyasen MalY oder MetC (EP1571223).

Die Steigerung des Transports von L-Cystein aus der Zelle ist eine weitere Möglichkeit, um die Produkt-Ausbeute im Medium zu erhöhen. Dies kann durch Überexpression sogenannter Efflux-Gene erreicht werden. Diese Gene kodieren für membrangebundene Proteine, die den Export von L-Cystein aus der Zelle vermitteln. Verschiedene Effluxgene für den L-Cystein-Export wurden beschrieben (US5972663A, US20440038352A1).

Der Export von L-Cystein aus der Zelle in das Fermentationsmedium hat mehrere Vorteile:
1) L-Cystein wird kontinuierlich aus dem intrazellulären Reaktionsgleichgewicht entzogen mit der Folge, dass der Spiegel dieser Aminosäure in der Zelle niedrig gehalten wird und somit die Feed-back-Inhibiton von sensitiven Enzymen durch L-Cystein unterbleibt:

   (1) L-Cystein (intrazellulär) ⇆ L-Cystein (Medium)
2) Das in das Medium ausgeschiedene L-Cystein wird in Gegenwart von Sauerstoff, der während der Kultivierung in das Medium eingebracht wird, zum Disulfid L-Cystin oxidiert (US5972663A):

   (2) 2 L-Cystein + 1/2 O₂ ⇆ L-Cystin + H₂O

   Da die Löslichkeit von L-Cystin in wässriger Lösung bei einem neutralen pH-Wert v.a. im Vergleich zu L-Cystein nur sehr gering ist, fällt das Disulfid schon bei einer niedrigen Konzentration aus und bildet einen weißen Niederschlag:

   (3) L-Cystin (gelöst) ⇆ L-Cystin (Niederschlag)

   Durch die Präzipitation von L-Cystin wird der Spiegel des im Medium gelösten Produkts abgesenkt, wodurch auch jeweils das Reaktionsgleichgewicht von (1) und (2) auf die Produktseite gezogen wird.
3) Der technische Aufwand für die Reinigung des Produkts ist bedeutend geringer, wenn die Aminosäure direkt aus dem Fermentationsmedium gewonnen werden kann, als wenn das Produkt intrazellulär akkumuliert und zuerst ein Zellaufschluss erfolgen muss.

Unter dem Begriff "Gesamtcystein" werden im Rahmen dieser Erfindung L-Cystein und die daraus gebildeten Verbindungen L-Cystin und Thiazolidin, die während der Fermentation entstehen und im Kulturüberstand und im Niederschlag akkumulieren, zusammengefasst.

Neben der gentechnischen Veränderung des L-Cystein-Produktions-stammes spielt auch die Optimierung des Fermentationsverfahrens, d.h. die Art und Weise der Kultivierung der Zellen, eine wichtige Rolle bei der Entwicklung eines effizienten Produktionsprozesses.

Dabei können verschiedene Kultivierungsparameter, wie z. B. die Art und Dosierung der Kohlenstoff- und Energiequelle, die Temperatur, die Versorgung mit Sauerstoff (DE102011075656A1), der pH-Wert sowie die Zusammensetzung des Kulturmediums, die Produktausbeute und/oder das Produktspektrum bei der fermentative Herstellung von L-Cystein beeinflussen.

Aufgrund laufend steigender Rohstoff- und Energiekosten besteht fortwährend der Bedarf, die Produkt-Ausbeute bei der L-Cystein-Herstellung zu steigern, um auf diese Weise die Wirtschaftlichkeit des Prozesses zu verbessern.

Aufgabe der vorliegenden Erfindung ist es, ein verbessertes Verfahren zur Herstellung von L-Cystein und seinen Derivaten L-Cystin und Thiazolidin mittels Fermentation eines Cystein-produzierenden Mikroorganismenstammes in einem Fermentationsmedium in einer Vorkultur und einer Hauptkultur in einem Fermenter zur Verfügung zu stellen. Die Aufgabe wird gelöst durch ein Verfahren, welches dadurch gekennzeichnet ist, dass dem Fermentationsmedium in der Hauptkultur L-Methionin, L-Isoleucin oder L-Threonin in einem Konzentrationsbereich von jeweils 0,5 bis 5 g/L zugesetzt werden.

In einem Screening von verschiedenen chemischen Substanzen bezüglich ihrer Wirkung auf den Produktionsprozess wurde überraschenderweise gefunden, dass durch Supplementation des Fermentationsmediums in der Hauptkultur mit den Aminosäuren L-Methionin, L-Isoleucin oder L-Threonin die Gesamtcystein-Ausbeute erhöht werden kann. Dies ist umso erstaunlicher, da die Bakterienstämme, die üblicherweise für einen Prozess zur fermentativen Cystein-Produktion eingesetzt werden, prototroph bezüglich der genannten Aminosäuren sind.

Eine positive Wirkung der Supplementation von L-Methionin, L-Isoleucin oder L-Threonin auf die fermentative Produktion von L-Cystein und den oben genannten Derivaten dieser Aminosäure in der Hauptkultur ist bisher nicht beschrieben oder nahegelegt worden. Dass durch den Zusatz dieser Aminosäuren die Ausbeute in einem fermentativen Cystein-Produktionsprozess gesteigert werden kann, ist vor allem deshalb überraschend, als keine dieser Aminosäuren eine metabolische Vorstufe bei der Cystein-Biosynthese darstellt.

L-Methionin, L-Isoleucin oder L-Threonin werden dem Fermentationsmedium in der Hauptkultur in einem Konzentrationsbereich von bevorzugt jeweils 0,5-5 g/L, besonders bevorzugt von jeweils 0,5-3 g/L zugesetzt.
Dabei sind die Kombinationen Methionin / Isoleucin und Methionin / Threonin gegenüber der Supplementation mit nur einer einzelnen Aminosäure bevorzugt. Besonders bevorzugt ist dabei die Kombination Methionin / Isoleucin. Bei der Zugabe einer Aminosäure-Kombination sind dieselben Konzentrationsbereiche bevorzugt wie für die einzelnen Aminosäuren angegeben.

Die Supplementierung des Mediums mit den entsprechenden Aminosäuren kann schon zu Beginn der Kultivierung der Hauptkultur als Batch-Zusatz erfolgen. Alternativ können die Aminosäuren auch erst während der Fermentation kontinuierlich dem Medium zudosiert werden.

Die Cystein-Produktion in der Hauptkultur (Produktionskultur) erfolgt prinzipiell nach einem dem Fachmann bekannten Fermentationsverfahren in einem Bioreaktor, vorzugsweise einem Rührkesselfermenter, mit einem Volumen von mindestens 5 m³, bevorzugt mindestens 20 m³, besonders bevorzugt mindestens 50 m³.

Die Kultivierung der Cystein-produzierenden Zellen wird typischerweise in mehreren Stufen durchgeführt. Ausgehend von einer Stammkultur erfolgt die Anzucht der Zellen in mindestens einer Vorkultur, bevor schließlich die Hauptkultur mit der letzten Vorkultur angeimpft wird. Dabei erfolgt in der Regel ein sukzessives Scale Up vom Schüttelkolben über Fermenter im Labormaßstab bis hin zum Bioreaktor im technischen Maßstab.

Als Mikroorganismen für das erfindungsgemäße Verfahren können alle im Stand der Technik beschriebenen Cystein-produzierenden Stämme eingesetzt werden. Derartige Stämme sind beispielsweise offenbart in US6218168B1, US5972663A, US20040038352A1, CA2386539A1, US20090053778 oder EP2138585.

Als Mikroorganismenstämme bevorzugt sind Vertreter aus der Familie der Enterobacteriaceae, besonders bevorzugt Vertreter der Gattungen *Eseherichia* und *Panfoea*, ganz besonders bevorzugt sind Stämme der Spezies *E. coli* und *P, ananatis*.

Von diesen Mikroorganismenstämmen sind wiederum Stämme bevorzugt, die entweder eine veränderte Serin-Q-Acetyl-Transferase besitzen, die im Vergleich zu dem entsprechenden Wildtypenzym eine um mindestens den Faktor 2 verminderte Feedback-Hemmung durch L-Cystein aufweist, oder die durch Überexpression eines Effluxgens einen um mindestens den Faktor 2 erhöhten Cystein-Export aus der Zelle im Vergleich zu einer Wildtypzelle aufweisen. Besonders bevorzugte Mikroorganismenstämme besitzen sowohl eine Serin-O-Acetyl-Transferase, die im Vergleich zu dem entsprechenden Wildtypenzym eine um mindestens den Faktor 2 verminderte Feedback-Hemmung durch L-Cystein aufweist, als auch einen durch Überexpression eines Effluxgens um mindestens den Faktor 2 erhöhten Cystein-Export aus der Zelle im Vergleich zu einer Wildtypzelle. Derartige Stämme sind beispielsweise aus US621S168B1 und US5972663A bekannt. Ganz besonders bevorzugte Stämme sind solche, die zusätzlich eine veränderte 3-Phosphoglycerat-Dehydrogenase mit einer im Vergleich zu dem entsprechenden Wildtypenzym um mindestens den Faktor 2 verminderten Feedback-Hemmung durch L-Serin besitzen (US2005009162A2) und bei denen mindestens ein L-Cystein-abbauendes Enzym soweit abgeschwächt ist, dass in der Zelle nur noch maximal 50 % dieser Enzym-Aktivität im Vergleich zu einer Wildtypzelle vorhanden ist.

Bevorzugte Varianten der Serin-O-Acetyl-Transferase weisen im Vergleich zu dem entsprechenden Wildtypenzym eine um mindestens den Faktor 5, besonders bevorzugt um mindestens den Faktor 10, ganz besonders bevorzugt um mindestens den Faktor 50 verminderte Feedback-Hemmung durch L-Cystein auf.

Das Effluxgen stammt vorzugsweise aus der Gruppe ydeD (siehe US5972663A), yfiK (siehe US20040038352A1), cydDC (siehe WO2004113373), bcr (siehe US2005221453) und emrAB (siehe US2005221453) von E. coli oder dem entsprechend homologen Gen aus einem anderen Mikroorganismus. Unter einem homologen Gen ist zu verstehen, dass die Sequenz dieses Gens zu mindestens 80 % mit der DNA-Sequenz des entsprechenden E. coli-Gens übereinstimmt.

Die Überexpression eines Effluxgens führt im Vergleich zu einer Wildtypzelle bevorzugt zu einem um mindestens den Faktor 5, besonders bevorzugt um mindestens den Faktor 10, ganz besonders bevorzugt um mindestens den Faktor 20 erhöhten Cystein-Export aus der Zelle.

Bevorzugte Varianten der 3-Phosphoglycerat-Dehydrogenase weisen im Vergleich zu dem entsprechenden Wildtypenzym eine um mindestens den Faktor 5, besonders bevorzugt um mindestens den Faktor 10, ganz besonders bevorzugt um mindestens den Faktor 50 verminderte Feedback-Hemmung durch L-Serin auf.

Das L-Cystein-abbauende Enzym stammt vorzugsweise aus der Gruppe Tryptophanase (TnaA) und Cystathionine-β-Lyase (MalY, MetC).

Besonders bevorzugt sind Mikroorganismenstämme, in denen mindestens eines dieser Enzyme soweit abgeschwächt ist, dass in der Zelle nur noch maximal 10 % der Enzym-Aktivität im Vergleich zu einem Wildtypstamm vorhanden ist. Ganz besonders bevorzugt sind Stämme, in denen mindestens eines dieser Enzyme völlig inaktiviert ist.

Die Kultivierung der Zellen bei der L-Cystein-Produktion wird unter aeroben Wachstumsbedingungen durchgeführt, wobei der Sauerstoff-Gehalt während der Fermentation in der Hauptkultur bei maximal 50 % Sättigung eingestellt wird. Die Regulation der Sauerstoff-Sättigung in der Kultur erfolgt dabei automatisch über die Gaszufuhr und die Rührgeschwindigkeit.

Als Kohlenstoffquelle dienen vorzugsweise Zucker, Zuckeralkohole, organische Säuren oder zuckerhaltige Pflanzenhydrolysate. Besonders bevorzugt werden im erfindungsgemäßen Verfahren als Kohlenstoffquelle Glucose, Fructose, Lactose, Glycerin oder Gemische, die zwei oder mehr dieser Verbindungen enthalten, eingesetzt.

Die Produktionsphase des erfindungsgemäßen Fermentationsverfahrens beginnt mit dem Zeitpunkt, ab dem erstmals L-Cystein, L-Cystin oder Thiazolidin in der Kulturbrühe der Hauptkultur nachgewiesen werden kann und dauert bis zum Ende der Kultivierung an. Typischerweise beginnt diese Phase ca. 8-10 h nach Inokulation des Produktionsfermenters.

Bevorzugt wird die Kohlenstoffquelle der Hauptkultur so zudosiert, dass der Gehalt der Kohlenstoffquelle im Fermenter während der Produktionsphase 10 g/l nicht übersteigt. Bevorzugt ist eine maximale Konzentration von 2 g/l, besonders bevorzugt von 0,5 g/l, ganz besonders bevorzugt von 0,1 g/l.

Als N-Quelle werden im erfindungsgemäßen Verfahren vorzugsweise Ammoniak, Ammoniumsalze oder Proteinhydrolysate verwendet. Bei Verwendung von Ammoniak als Korrekturmittel zur pH-Statisierung wird während der Fermentation regelmäßig diese N-Quelle nachdosiert.

Als weitere Medienzusätze können Salze der Elemente Phosphor, Chlor, Natrium, Magnesium, Stickstoff, Kalium, Calcium, Eisen und in Spuren (d.h. in µM Konzentrationen) Salze der Elemente Molybdän, Bor, Kobalt, Mangan, Zink und Nickel zugesetzt werden.

Des Weiteren können organische Säuren (z.B. Acetat, Citrat) und Vitamine (z.B. B1, B6) dem Medium zugesetzt werden.

Als komplexe Nährstoffquellen können z.B. Hefeextrakt, Maisquellwasser, Sojamehl oder Malzextrakt zum Einsatz kommen.

Die Inkubationstemperatur für mesophile Mikroorganismen wie z.B. *E. coli* oder *P. ananatis* beträgt vorzugsweise 15 - 45 °C, besonders bevorzugt 30 - 37 °C.

Der pH-Wert des Fermentationsmediums liegt während der Fermentation bevorzugt im pH-Bereich von 5,5 bis 7,5, besonders bevorzugt ist ein pH-Wert von 6,5-7,5, ganz besonders bevorzugt ist ein pH-Wert von 7,0.

Für die Herstellung von L-Cystein und L-Cystein-Derivaten muss während der Fermentation eine Schwefelquelle zugefüttert werden. Bevorzugt kommen dabei Sulfate oder Thiosulfate zum Einsatz.

Mikroorganismen, die nach dem beschriebenen Verfahren fermentiert werden, sezernieren in einem Batch- oder Fedbatch-Prozess nach einer Anwachsphase in einem Zeitraum von acht bis 150 Stunden L-Cystein und davon abgeleitete Verbindungen in hoher Effizienz in das Fermentationsmedium.

Nach der Fermentation kann das als Niederschlag vorliegende L-Cystin nach bekannten Verfahren von den restlichen Bestandteilen der Kulturbrühe beispielsweise mit Hilfe eines Dekanters abgetrennt werden.

Zur weiteren Aufreinigung des Rohprodukts können folgende Schritte durchgeführt werden:
- Lösen des Rohproduktes mit einer Mineralsäure
- Klären der Rohproduktlösung durch Zentrifugation oder Filtration
- Entfärbung der Lösung
- Fällungskristallisation

Die Reduktion von L-Cystin zu L-Cystein kann beispielsweise über einen elektrochemischen Prozess, wie in EP0235908 beschrieben, erfolgen.

Die folgenden Beispiele dienen der weiteren Erläuterung der Erfindung.

### Beispiel 1: Erzeugung von Cystein-Produktionsstämmen

Es wurden die Wildtyp-Stämme *E. coli* W3110 (ATCC 27325) und *P. ananatis* (ATCC 11530) jeweils mit dem Plasmid pACYC184/cysEX-GAPDH-ORF306 (offenbart in Beispiel 2 von US5972663A) mittels Elektroporation wie in US5972663A beschrieben transformiert. Das Plasmid pACYC184/cysEX-GAPDH-ORF306 enthält neben dem Replikationsursprung und einem Tetrarcyclin-Resistenzgen auch noch das cysEX-Allel, das für eine Serin-O-Acetyl-Transferase mit einer verminderten Feedback-Hemmung durch L-Cystein kodiert sowie das Effluxgen ydeD (ORF306), dessen Expression durch den konstitutiven GAPDH-Promotor gesteuert wird.
Die Selektion auf Plasmid-tragende Zellen erfolgte auf ALB-Agarplatten, die 15 mg/l Tetracyclin enthielten.
Nach einer erneuten Plasmidisolierung mittels dem QIAprep Spin Plasmid Kit (Qiagen GmbH) und einer Pestriktionsanalyse wurden die gewünschten Transformanden, d.h. Zellen, die das Plasmid pACYC184/cysEX-GAPDH-ORF306 aufgenommen haben, isoliert und in die Kultivierung eingesetzt, welche in den Beispielen 2 und 3 beschrieben ist.

### Beispiel 2: Cystein-Produktion mit Supplementation verschiedener Aminosäuren

### Vorkultur 1:

20 ml LB-Medium mit 15 mg/l Tetracyclin wurden in einem Erlenmeyerkolben (100 ml) mit dem jeweiligen Stamm (*E. coli* W3110 pACYC184/cysEX-GAPDH-ORF306 bzw. *P. ananatis* pACYC184/cysEX-GAPDH-ORF306) beimpft und für sieben Stunden auf einem Schüttler (150 rpm, 30 °C) inkubiert.

### Vorkultur 2:

Anschließend wurde die Vorkultur 1 vollständig in 100 ml SM1-Medium überführt (12 g/l K₂HPO₄, 3 g/l KH₂PO₄, 5 g/l (NH₄)₂SO₄, 0,3 g/l MgO₄ x 7 H₂O, 0,015 g/l CaCl₂ x 2 H₂O, 0,002 g/l FeSO₄ x 7 H₂O, 1 g/l Na₃Citrat x 2 H₂4, 0,1 g/l NaCl, 1 ml/l Spurenelementlösung, bestehend aus 0,15 g/l Na₂MoO₄ x 2H₉O, 2,5 g/ H₃BO₃, 0,7 g/l CoCl₂ x 6 H₂O, 0,25 g/l CuSO₄ x 5 H₂O, 1,6 g/l MnCl₂ x 4 H₂O, 0,3 g/l ZnSO₄ x 7 H₂O), das mit 5 g/l Glucose, 5 mg/l Vitamin B1 und 15 mg/l Tetracyclin supplementiert war. Die Kulturen wurden in Erlenmeyerkolben (1 1) bei 30 °C für 17 h mit 150 rpm geschüttelt. Nach dieser Inkubation lag die optische Dichte bei 600 nm (OD₆₀₀) zwischen 3 und 5.

### Hauptkultur:

Die Fermentation wurde in Fermentern des Typs BIOSTAT B der Firma Sartorius Stedim durchgeführt. Es wurde ein Kulturgefäß mit 2 1 Gesamtvolumen verwendet. Das Fermentationsmedium (900 ml) enthält 15 g/l Glucose, 10 g/l Trypton (Difco), 5 g/l Hefeextrakt (Difco), 5 g/l (NH₄)₂SO₄, 1,5 g/L KH₂PO₄, 0,5 g/l NaCl, 0,3 g/l MgOSO₄ x 7 H₂O, 0,015 g/l CaCl₂ x 2 H₂O, 0,075 g/l FeSO₄ x 7 H₂O, 1 g/l Na₃Citrat x 2 H₂O und ml Spurenelementlösung (siehe oben), 0,005 g/l Vitamin B1 und 15 mg/l Tetracyclin. In verschiedenen Versuchsanstellungen wurden dem Medium außerdem noch die Aminosäuren L-Methionin, L-Isoleucin oder L-Threonin zugesetzt, entweder einzeln oder in Kombinationen in verschiedenen Konzentrationen (s. Tabelle 1).
Der pH-Wert im Fermenter wurde zu Beginn durch Zupumpen einer 25% NH₄OH-Lösung auf 7,0 eingestellt. Während der Fermentation wurde der pH-Wert durch automatische Korrektur mit 25% NH₄OH auf einem Wert von 7,0 gehalten. Zum Animpfen wurden 100 ml der Vorkultur 2 in das Fermentergefäß gepumpt. Das Anfangsvolumen betrug somit etwa 1 1. Die Kulturen wurden zu Beginn mit 400 rpm gerührt und mit 2 vvm einer über einen Sterilfilter entkeimten Druckluft begast. Unter diesen Startbedingungen war die Sauerstoff-Sonde vor der Inokulation auf 100% Sättigung kalibriert worden. Der Soll-Wert für die O₂-Sättigung während der Fermentation wurde auf 50 % eingestellt. Nach Absinken der O₂-Sättigung unter den Soll-Wert wurde eine Regulationskaskade gestartet, um die O₂-Sättigung wieder an den Soll-Wert heranzuführen. Dabei wurde zunächst die Gaszufuhr kontinuierlich erhöht (auf max. 5 vvm) und anschließend die Rührgeschwindigkeit (auf max. 1.500 rpm) kontinuierlich gesteigert.
Die Fermentation wurde bei einer Temperatur von 30 °C durchgeführt. Nach 2 h Fermentationszeit erfolgte die Zufütterung einer Schwefelquelle in Form einer sterilen 60 % Natrium-Thiosulfat x 5 H₂O - Stammlösung mit einer Rate von 1,5 ml pro Stunde. Sobald der Glukose-Gehalt im Fermenter von anfänglich 15 g/l auf ca. 2 g/l abgesunken war, erfolgte eine kontinuierliche Zudosierung einer 56% Glukose-Lösung. Die Fütterungsrate wurde so eingestellt, dass die Glukosekonzentration im Fermenter 2 g/l fortan nicht mehr überstieg. Die Glukose-Bestimmung wurde mit einem Glukoseanalysator der Firma YSI (Yellow Springs, Ohio, USA) durchgeführt.
Die Fermentationsdauer betrug 48 Stunden. Danach wurden Proben entnommen und der Gehalt von L-Cystein und den davon abgeleiteten Derivaten im Kulturüberstand (v.a. L-Cystein und Thiazolidin) und im Niederschlag (L-Cystin) jeweils getrennt voneinander bestimmt (s. Tabelle 1). Zu diesem Zweck wurde jeweils der colorimetrische Test von Gaitonde verwendet (Gaitonde, M. K. (1967), Biochem. J. 104, 627-633). Dabei ist zu berücksichtigen, dass der Test unter den stark sauren Reaktionsbedingungen nicht zwischen L-Cystein und dem in EP0885962 A1 beschriebenen Kondensationsprodukt von L-Cystein und Pyruvat, der 2-Methyl-thiazolidin-2,4-dicarbonsäure (Thiazolidin), diskriminiert. L-Cystin, das durch Oxidation zweier L-Cystein-Molekülen entsteht, wird im Test durch Reduktion mit Dithiothreitol in verdünnter Lösung bei pH 8,0 ebenfalls als L-Cystein nachgewiesen. Das im Niederschlag befindliche L-Cystin musste zuerst in 8% Salzsäure aufgelöst werden, bevor es auf dieselbe Art quantifiziert werden konnte.

**Tabelle 1: Gehalt von L-Cystein und L-Cystein-Derivaten in der Kulturbrühe nach 48 h**

| **Aminosäure-Zusatz [g/L]** | **Cystein-Gehalt [g/L] nach 48 h** | | | | | |
|---|---|---|---|---|---|---|
| | ***E. coli*** | | | ***P. ananatis*** | | |
| | Überstand¹ | Niederschlag² | Gesamtcystein³ | Überstand¹ | Niederschlag² | Gesamtcystein³ |
| - | 6,1 | 10,8 | **16,9** | 3,4 | 7,6 | **11,0** |
| **Met** | 6,2 | 11,3 | **17,5** | 3,5 | 8,4 | **11,9** |
| **1,5** | | | | | | |
| **Ile** | 6,5 | 11,3 | **17,8** | 3,7 | 8,0 | **11,7** |
| **1,5** | | | | | | |
| **Thr** | 6,3 | 11,0 | **17,3** | 3,5 | 8,3 | **11,8** |
| **1,5** | | | | | | |
| **Met / Ile** | 6,8 | 13,1 | **19,9** | 4,0 | 9,2 | **13,2** |
| **0,1 / 0,1** | | | | | | |
| **Met / Ile** | 7,1 | 14,4 | **21,5** | 4,2 | 10,5 | **14,7** |
| **0,5 / 0,5** | | | | | | |
| **Met / Ile** | 7,3 | 16,3 | **23,6** | 4,1 | 11,2 | **15,3** |
| **3 / 3** | | | | | | |
| **Met / Ile** | 7,5 | 16,0 | **23,5** | 4,3 | 11,0 | **15,3** |
| **10 / 10** | | | | | | |
| **Met / Thr** | 6,8 | 12,2 | **19,0** | 3,9 | 9,0 | **12,9** |
| **0,1 / 0,1** | | | | | | |
| **Met / Thr** | 7,2 | 13,6 | **20,8** | 4,2 | 9,8 | **14,0** |
| **0,5 / 0,5** | | | | | | |
| **Met / Thr** | 7,2 | 14,9 | **22,1** | 4,0 | 11,0 | **15,0** |
| **3 / 3** | | | | | | |
| **Met / Thr** | 7,4 | 14,6 | **22,0** | 4,6 | 10,3 | **14,9** |
| **10 / 10** | | | | | | |

| | | | | | | |
|---|---|---|---|---|---|---|
| **¹: Summe des im Überstand gelösten L-Cysteins, L-Cystins und Thiazolidins** **²: L-Cystin im Niederschlag** **³: Gesamtcystein (Summe von Überstand und Niederschlag)** | | | | | | |

## Patentansprüche

1. Verfahren zur Herstellung von L-Cystein und seinen Derivaten L-Cystin und Thiazolidin mittels Fermentation eines Cystein-produzierenden Mikroorganismenstammes aus der Familie der Enterobacteriaceae in einem Fermentationsmedium in einer Vorkultur und einer Hauptkultur in einem Fermenter, welches **dadurch gekennzeichnet ist, dass** dem Fermentationsmedium in der Hauptkultur L-Methionin, L-Isoleucin oder L-Threonin in einem Konzentrationsbereich von jeweils 0,5 bis 5 g/L zugesetzt werden.

2. Verfahren nach Anspruch 1, **dadurch gekennzeichnet, dass** Methionin und Isoleucin oder Methionin und Threonin zugesetzt werden.

3. Verfahren nach Anspruch 2, **dadurch gekennzeichnet, dass** Methionin und Isoleucin zugesetzt werden.

4. Verfahren nach einem der Ansprüche 1 bis 3, **dadurch gekennzeichnet, dass** der Mikroorganismenstamm entweder eine veränderte Serin-O-Acetyl-Transferase besitzt, die im Vergleich zu einem entsprechenden Wildtypenzym eine um mindestens den Faktor 2 verminderte Feedback-Hemmung durch L-Cystein aufweist, oder der durch Überexpression eines Effluxgens einen um mindestens den Faktor 2 erhöhten Cystein-Export aus der Zelle im Vergleich zu einer Wildtypzelle aufweist.

5. Verfahren nach Anspruch 1 bis 4, **dadurch gekennzeichnet, dass** der Mikroorganismenstamm sowohl eine Serin-O-Acetyl-Transferase, die im Vergleich zu dem entsprechenden Wildtypenzym eine um mindestens den Faktor 2 verminderte Feedback-Hemmung durch L-Cystein aufweist, als auch einen durch Überexpression eines Effluxgens um mindestens den Faktor 2 erhöhten Cystein-Export aus der Zelle im Vergleich zu einer Wildtypzelle aufweist, sowie zusätzlich eine veränderte 3-Phosphoglycerat-Dehydrogenase mit einer im Vergleich zu dem entsprechenden Wildtypenzym um mindestens den Faktor 2 verminderten Feedback-Hemmung durch L-Serin besitzt und bei dem mindestens ein L-Cystein-abbauendes Enzym soweit abgeschwächt ist, dass in der Zelle nur noch maximal 50 % dieser Enzym-Aktivität im Vergleich zu einer Wildtypzelle vorhanden ist.

6. Verfahren nach einem der Ansprüche 1 bis 5, **dadurch gekennzeichnet, dass** die Kultivierung der Zellen bei der L-Cystein-Produktion unter aeroben Wachstumsbedingungen durchgeführt wird, wobei der Sauerstoff-Gehalt während der Fermentation bei maximal 50 % Sättigung eingestellt wird.

7. Verfahren nach einem der Ansprüche 1 bis 6, **dadurch gekennzeichnet, dass** eine Kohlenstoffquelle so zudosiert wird, dass der Gehalt der Kohlenstoffquelle im Fermentationsmedium während der Produktionsphase 10 g/l nicht übersteigt.

## Claims

1. Method for the production of L-cysteine and its derivatives L-cystine and thiazolidine by means of fermentation of a cysteine-producing microorganism strain of the Enterobacteriaceae family in a fermentation medium in a preculture and a main culture in a fermenter, which is **characterized in that** L-methionine, L-isoleucine or L-threonine are added to the fermentation medium in the main culture each in a concentration range from 0.5 to 5 g/l.

2. Method according to Claim 1, **characterized in that** methionine and isoleucine or methionine and threonine are added.

3. Method according to Claim 2, **characterized in that** methionine and isoleucine are added.

4. Method according to any one of Claims 1 to 3, **characterized in that** the microorganism strain either possesses a modified serine-O-acetyl transferase which, in comparison with a corresponding wild type enzyme, has a feedback inhibition due to L-cysteine decreased by least the factor 2, or which has a cysteine export out of the cell increased by at least the factor 2 in comparison with a wild type cell by overexpression of an efflux gene.

5. Method according to Claim 1 to 4, **characterized in that** the microorganism strain not only has a serine-O-acetyl transferase which, in comparison with the corresponding wild type enzyme, has a feedback inhibition due to L-cysteine decreased by at least the factor 2, but also has a cysteine export out of the cell increased by at least the factor 2 in comparison with a wild type cell by overexpression of an efflux gene, and also, in addition, a modified 3-phosphoglycerate dehydrogenase having a feedback inhibition due to L-serine decreased by at least the factor 2 in comparison with the corresponding wild type enzyme and in which at least one L-cysteine-degrading enzyme is attenuated so far that, in the cell, only a maximum of 50% of this enzyme activity is present in comparison with a wild type cell.

6. Method according to any one of Claims 1 to 5, **characterized in that** the cells are cultured in the case of L-cysteine production under aerobic growth conditions, wherein the oxygen content during the fermentation is set at a maximum 50% saturation.

7. Method according to any one of Claims 1 to 6, **characterized in that** a carbon source is added in such a manner that the content of the carbon source in the fermentation medium does not exceed 10 g/l during the production phase.

## Revendications

1. Procédé pour la préparation de L-cystéine et de ses dérivés L-cystine et thiazolidine par fermentation d'une souche de micro-organismes produisant de la cystéine de la famille des entérobactéries dans un milieu de fermentation, dans une préculture et une culture principale, dans un fermenteur, qui est **caractérisé en ce que** le milieu de fermentation est additionné, dans la culture principale, de L-méthionine, de L-isoleucine ou de L-thréonine dans une plage de concentration à chaque fois de 0,5 à 5 g/l.

2. Procédé selon la revendication 1, **caractérisé en ce qu'**on ajoute de la méthionine et de l'isoleucine ou de la méthionine et de la thréonine.

3. Procédé selon la revendication 2, **caractérisé en ce qu'**on ajoute de la méthionine et de l'isoleucine.

4. Procédé selon l'une quelconque des revendications 1 à 3, **caractérisé en ce que** la souche de micro-organismes possède, soit une sérine-O-acétyl-transférase modifiée, qui présente une rétro-inhibition par la L-cystéine diminuée d'au moins un facteur 2 par rapport à l'enzyme de type sauvage correspondante, soit une excrétion de cystéine hors de la cellule augmentée d'au moins un facteur 2 par rapport à une cellule de type sauvage par surexpression d'un gène d'efflux.

5. Procédé selon la revendication 1 à 4, **caractérisé en ce que** la souche de micro-organismes possède une sérine-O-acétyl-transférase, qui présente une rétro-inhibition par la L-cystéine diminuée d'au moins un facteur 2 par rapport à l'enzyme de type sauvage correspondante, ainsi qu'une une excrétion de cystéine hors de la cellule augmentée d'au moins un facteur 2 par rapport à une cellule de type sauvage par surexpression d'un gène d'efflux, ainsi qu'en outre une 3-phosphoglycérate-déshydrogénase modifiée, présentant une rétro-inhibition par la L-sérine diminuée d'au moins un facteur 2 par rapport à l'enzyme de type sauvage et dans laquelle au moins une enzyme de dégradation de la L-cystéine est atténuée de manière telle que la cellule ne présente plus qu'au maximum 50% de cette activité enzymatique par rapport à une cellule de type sauvage.

6. Procédé selon l'une quelconque des revendications 1 à 5, **caractérisé en ce que** la culture des cellules lors de la production de L-cystéine est réalisée dans des conditions de croissance aérobies, la teneur en oxygène étant réglée au maximum à une saturation 50% lors de la fermentation.

7. Procédé selon l'une quelconque des revendications 1 à 6, **caractérisé en ce qu'**on ajoute en dosant une source de carbone de manière telle que la teneur en source de carbone dans le milieu de fermentation ne dépasse pas 10 g/l pendant la phase de production.
